COMPLETE DOCUMENT

(19) Europäisches Patentamt
European Patent Office
Office européen des brevets

(11) Publication number: 0 066 552
A1

(12) EUROPEAN PATENT APPLICATION

(21) Application number: 82830126.7

(22) Date of filing: 10.05.82

(51) Int. Cl.$^3$: C 12 Q 1/34
C 12 Q 1/00, G 01 N 33/70

(30) Priority: 28.05.81 IT 2200981

(43) Date of publication of application:
08.12.82 Bulletin 82/49

(84) Designated Contracting States:
AT BE CH DE FR GB LI NL SE

(71) Applicant: CHEMICAL LABORATORIES S.r.l.
Via Nicola d'Apulia, 15
I-20125 Milano(IT)

(72) Inventor: Torelli, Giorgio
Via Nicola d'Apulia 15
Milano(IT)

(74) Representative: Cicogna, Franco et al,
Ufficio Internazionale Brevetti Dott.Prof. Franco Cicogna
Via Visconti di Modrone, 14/A
I-20122 - Milano(IT)

(54) **Method for determining the creatinine concentration in biological liquids and reactive for carrying out the method.**

(57) The method for determining the creatinine concentration or contents in biological liquids comprises the steps of transforming enzymatically the creatinine into glycine, acetaldehyde and hydrogen peroxide and proportioning one of these compounds.

Advantageously there is proportioned the hydrogen peroxide by titration, or potentiometric, colorimetric or enzymatic procedures. Preferably the enzymatic method is used by catalasis or peroxydasis in the presence of such compounds effective to provide a chromogen compound. The enzymatic transformation of creatinine into the three compounds is carried out by means of creatinineamidohydrolasis, creatineamidohydrolasis and sarcosineoxydasis.

EP 0 066 552 A1

Croydon Printing Company Ltd.

TITLE
see front page

BACKGROUND OF THE INVENTION

The present invention relates to a method for determining the creatinine contents in biological liquids and to a reagent for carrying out the method.

As it is well known,the creatinine contents in biological liquids is conventionally determined by means of the Jaffé reaction (see Jaffé M-Zschr.-Physiol Chem.10.391 1886) which is based on the creatinine property of reducing the picric and picramic acids in an alkaly solution and providing a red-orange solution.More specifically the concentration of the reaction product is determined by colorimetry at a wave length of about 510nm. The analysis can be carried out at equilibrium conditions (end point) either by means of a preliminary deproteinizing step or without using any steps of this type,and under kinetic conditions (fixed time) without any deproteinizing steps.

A drawback which is common to all of these methods is the inherent lack of specificity of the chemical process,since the reduction of picric acid to picramic acid is not a characteristic peculiar to creatinine and,therefore,it may also be

caused by other substances present in biological liquids, such as many metabolism compounds related to creatinine .

Thus the data reproductibility based on methods using the same principle is a very poor one.

SUMMARY OF THE INVENTION

Accordingly the task of the present invention is to obviate the mentioned drawback by providing a new method for determining the creatinine contents or concentration in biological liquids effective to allow for a high reaction specificity to be obtained as well as highly reliable results about the creatinine amount contained in said biological liquids.

Within the scope of the mentioned task, it is a primary object of the present invention to provide a method for determining the creatinine contents or concentration in biological liquids which is effective to use a single reagent, thereby affording the possibility of easily automatizing the procedure to be followed.

Yet another object of the present invention is to provide a method for determining the creatinine contents or concentration in biological liquids

effective to give a high reproductibility of the analytical data.

Yet another object of the present invention is to provide a reagent for determining the creatinine contents or concentration in biological liquids which is effective to carry out the above mentioned method.

According to one aspect of the present invention the mentioned task and objects as well as yet other objects which will become more apparent thereinafter are achieved by a method for determining the creatinine contents or concentration in biological liquids characterized in that it comprises the steps of enzymatically transforming creatinine to glycine,acetaldehyde and hydrogen peroxide and proportioning one of the reaction end products.

Advantageously the proportioned product is the hydrogen peroxide,which is proportioned by using a conventional proportioning method,such as titration,potentiometric methods,colorimetric methods or enzymatic methods or the like.

Preferably said hydrogen peroxide is enzymatically proportioned,by using catalasis or peroxydasis in the presence of compounds effective

to provide a chromogen compound which is colorimetrically analyzed.

Advantageously the enzymes used in the instant method for determining the creatinine contents or concentration consist of the creatinine-amidohydrolasis effective to transform creatinine into creatine by addition of water, the creatine-amidohydrolasis effective to transform creatine into urea and sarcosine by addition of water and the sarcosineoxydasis which, also by addition of water, is effective to transform sarcosine into glycine, acetaldehyde and hydrogen peroxide.

In particular the compounds effective to provide a chromogen compound upon catalasis or peroxydasis are 4-aminophenazone and the like, phenol and the halogenated derivavives thereof, or aromatic amines.

The reagent preferably comprises a phosphate buffer, aminoantipirine, dichlorophenolsulphonate, creatinineamidohydrolasis, creatineamidohydrolasis, sarcosineoxydasis and peroxydasis.

## BRIEF DESCRIPTION OF THE DRAWINGS

The invention will be described in a more detailed way thereinafter, with reference to the

accompanying drawings, where:

Fig.1 is a graph of the creatinine contents or concentration versus the absorbancy;and

Fig.2 is a graph illustrating the data obtained by the method according to the invention and compared against those obtained by the conventional deproteinizing Jaffé method.

## DESCRIPTION OF THE PREFERRED EMBODIMENTS

With reference to the figures of the accompanying drawings, the method for enzymatically determining the creatinine contents or concentration in biological liquids according to the present invention is based on the following reaction relationships:

$$\text{Creatinine} + H_2O \xrightleftharpoons{\text{Creatinineamidohydrolasis}} \text{Creatine}$$

$$\text{Creatine} + H_2O \xrightleftharpoons{\text{Creatineamidohydrolasis}} \text{Urea} + \text{Sarcosine}$$

$$\text{Sarcosine} + H_2O \xrightleftharpoons{\text{Sarcosineoxydasis}} \text{Glycine} + \text{Acetaldehyde} + H_2O_2$$

In principle, in order to determine the creatinine amount in the biological liquid, it is necessary to proportion one of the three reaction products, that is glycine, acetaldehyde or hydrogen peroxide.

Preferably, due to practical reasons, there is determined the hydrogen peroxide amount, for example by using conventional quantitative analytical methods, such as titration, potentiometric procedures, colorimetric and enzymatic methods and so on.

The most preferred way is the enzymatic procedure, by using catalasis or peroxydasis which, in the presence of 4-aminophenazone or the like, phenol and halogenated derivatives thereof, aromatic amines, are effective to provide a chromogen compound having a maximum at about 510nm. These reactions are generally indicated as of the "Trinder" type (see Trinder P.J.Clin.Pat.22.158 (1969) ).

Among the phenol halogenated derivatives 2,4-dichlorophenol, 2,4-dichlorophenolsulphonate, 2,6-dichlorophenol, 2-bromophenol, 2-chlorophenol, tribromophenol, 2,4-dibromophenol, 2,4-dibromophenol-sulphanate, hydroxybenzoate have been found to be particularly effective.

Among aromatic amines, aniline, dimethylaniline, diethylaniline have been found to be particularly effective.

A particularly effective method for determining the hydrogen peroxide amount is that exploiting the property of said hydrogen peroxide or oxidizing

iodides, such as potassium iodide, to iodine which is determined colorimetrically at 365 or 405nm (or by filters near to this wavelenght).

A very effective method for determining the $H_2O_2$ amount is obtained by the action of peroxidasis on a mixture of 3 hydroxy 2-4-6-tri-iodine benzoic acid and 3-methyl-benzothioazoline-hydrazone-hydrochloride monohydrate.

An example of a suitable reagent for carrying out the abovementioned reactions will be described thereinbelow.

REAGENT

| | | | | | |
|---|---|---|---|---|---|
| - Aminoantipirine | from | 0.10 | to | 1mmol/l | |
| - 3 hydroxy 2-4-6 triiodine benzoic acid | " | 3 | " | 50 | " |
| - Creatinineamido-hydrolasis | " | 1,000 | " | 40,000 | U/l |
| - Creatineamido-hydrolasis | " | 1,000 | " | 20,000 | " |
| - Sarcosineoxydasis | " | 200 | " | 3,000 | " |
| - Peroxydasis | " | 300 | " | 3,000 | " |
| - 3-methyl-2-benzothio-azolinone-hydrazone-hydro-chloride monohydrate | " | 2 | " | 30 | $\mu$mol/l |

The phosphate buffer may consist of 12g/l of monopotassium phosphate, 19.5 g/l of bipotassium phosphate, 4g/l of tetrasodium EDTA and 5g/l of triton-100.

The following is a preferred formulation:

| | | |
|---|---|---|
| - Phosphate buffer | 100 | mmol/l |
| - Aminoantipirine | 0.30 | " |
| - 3-hydroxy 2-4-6 triiodine-benzoic acid | 1 | " |
| - Creatinineamidohydrolasis | 16,000 | U/l |
| - Creatineamidohydrolasis | 8,000 | " |
| - Sarcosineoxydasis | 1,000 | " |
| - Dipotassium EDTA | 4 | g/l |
| - 3-methyl-2-benzothioazolinone-hydrochloride monohydrate | 8 | μmol/l |
| - Potassium and ferrcyanide | 30 | " |
| - Surfactants and antibacteria agents | | |

In order to carry out the determining procedure, 50μl of standard and sample are diluted with 2ml reagent.

Then the mixture is introduced into a thermostat adjusted at 37°C for 45 minutes and a read out is performed at 560 nm against the respective blanks obtained by diluting 50μl of standard and

samples with the same reagent with the exception that the latter did not contain creatinineamidohydrolasis.

The sample creatinine is calculated by using the following formula:

$$\text{sample creatinine (mg/dl)} = \frac{\text{sample absorbancy}}{\text{standard absorbancy}} \times \text{standard concentration}$$

Thus the methods according to the invention allows for a great reaction specificity to be obtained by using a single reactive, which permits to easily mechanize the procedure and affords highly reproducible data. In addition to the foregoing, as it will be clear from the graph of fig.1, the advantage is obtained of having a linear reaction up to a creatinine concentration of 8mg/dl, which value is an effectively suitable one for the examined creatinine containing biological liquids. Another advantage consists of the standard reproductibility which, as carried out on 20 samples, has been found to be of 2.9%, while the serum reproductibility, also on 20 samples, was of 4.1%.

In the graph of fig.2 the data obtained by the method according to the invention has been indicated with respect to the data determined by the conventional

deproteinizing Jaffé method.It is possible to recognize easily that the high correlation (r=0.96) defines a good reliability for the method according to the present invention.

# C L A I M S

1- A method for determining the creatinine contents or concentration in biological liquids characterized in that it comprises the steps of enzymatically transforming creatinine to glycine, acetaldehyde and hydrogen peroxide and proportioning one of the reaction end products.

2- A method according to claim 1, characterized in that it comprises the step of proportioning said hydrogen peroxide by means of the conventional analytical procedures, such as titration, potentiometric methods, colorimetric or enzymatic methods and the like.

3- A method according to claim 2, characterized in that said hydrogen peroxide is proportioned enzymatically, by using catalasis or peroxydasis in the presence of compounds giving a chromogen compound effective to be colorimetrically analyzed.

4- A method according to claim 1, characterized in that the enzymes used in said method for determining the creatinine contents or concentration consist of creatinineamidohydrolasis effective to transform the creatinine into creatine by addition of water,

creatineamidohydrolasis effective to transform creatine into urea and sarcosine by addition of

water and sarcosineoxydasis which, by adding water thereto, is effective to transform sarcosine into glycine, acetaldehyde and hydrogen peroxide.

5- A method according to claim 3, characterized in that said compounds providing, upon catalasis or peroxydasis, said chromogen compound consist of 4-aminophenazone and the like, phenol or halogenated derivavives thereof, or aromatic amines.

6- A method according to claim 5, characterized in that the phenol halogenated derivatives used for forming said chromogen compound consist of 2,4-dichlorophenol, 2,4-dichlorophenolsulfanate, 2,6-dichlorophenol, 2-bromophenol, 2-chlorophenol, tribromophenol, 2,4-dibromophenol, 4-dibromophenolsulfanate, hydroxybenzoate and so on, and in that said aromatic amines consist of aniline, dimethylaniline, diethylaniline.

7- A method according to claim 5, characterized in that said compounds effective to provide, upon catalasis or peroxydasis, said chromogen compound consist of 3-hydroxy-3-5-didhloro-sodium benzenesulphamate acid with 3-methyl-2-benzothioazolinonehydrazone-hydrochloride monohydrate and the like.

8- A reagent for carrying out a method according

to claims 1 to 7, characterized in that it comprises a phosphate buffer, creatineaminohydrolasis, sarcosineoxydasis and peroxydasis.

FIG. 1

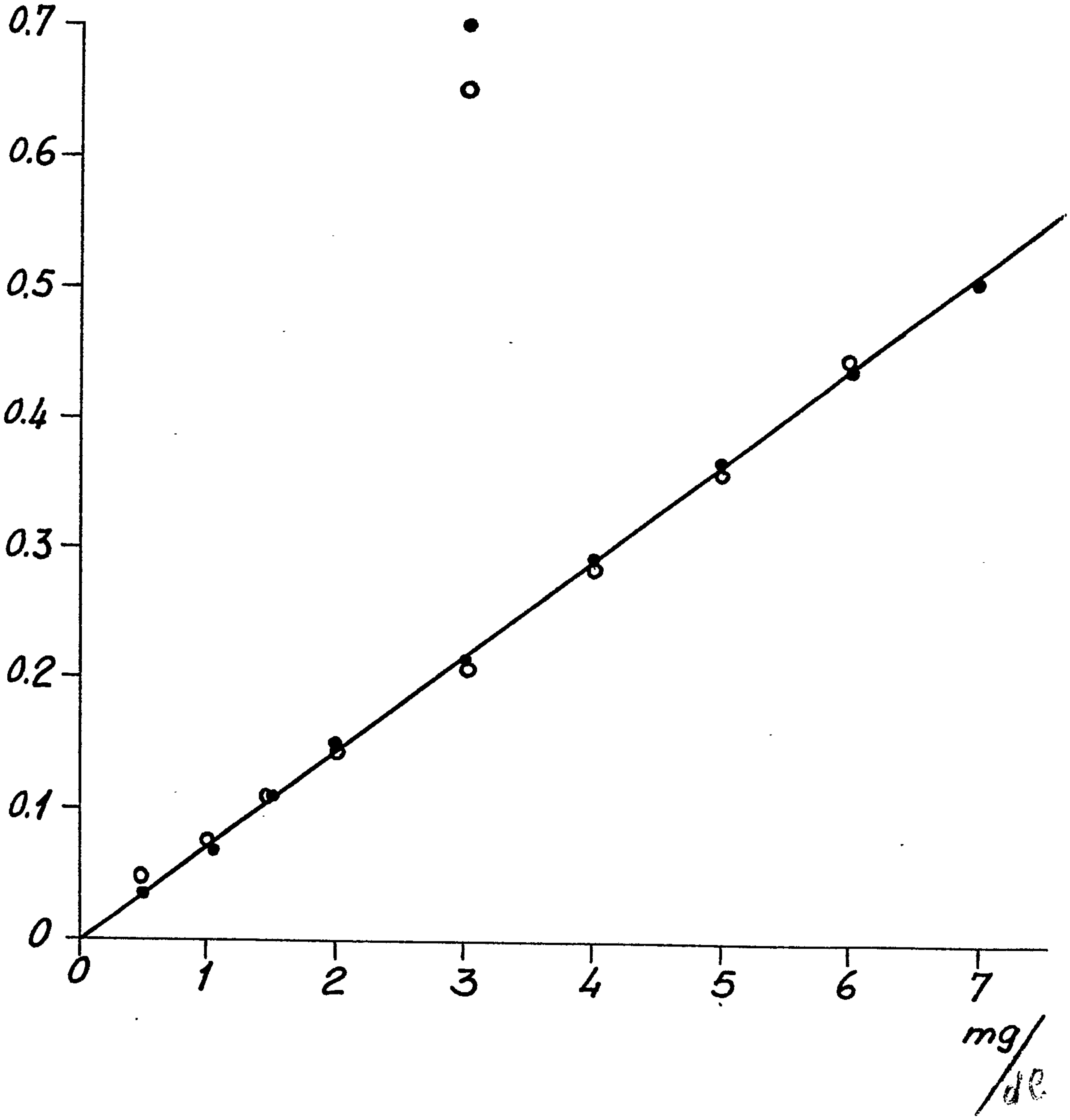

FIG. 2

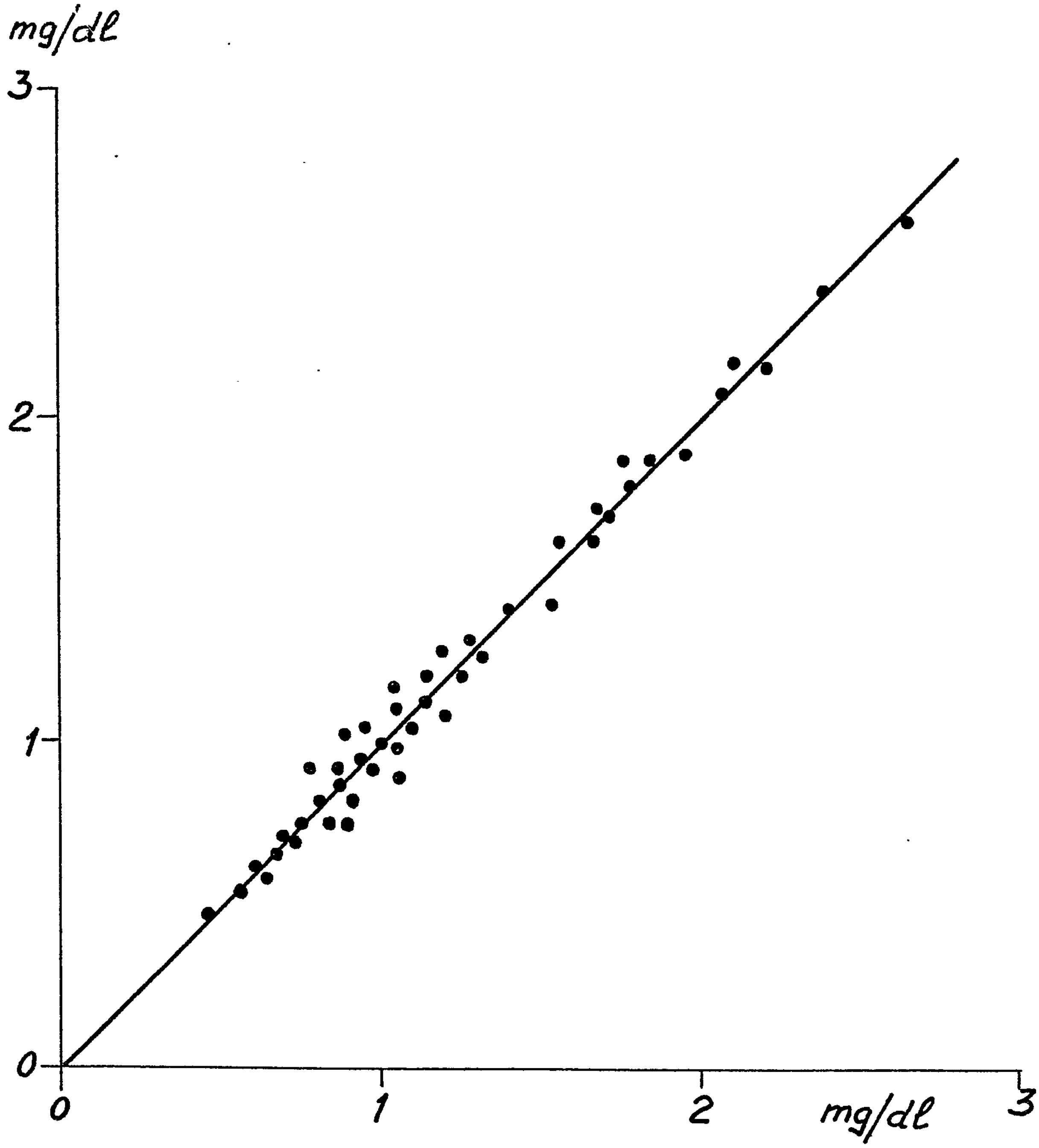

0066552

European Patent Office

# EUROPEAN SEARCH REPORT

Application number
EP 82 83 0126.7

| Category | Citation of document with indication, where appropriate, of relevant passages | Relevant to claim | CLASSIFICATION OF THE APPLICATION (Int. Cl. 3) |
|---|---|---|---|
| X | CHEMICAL AND PHARMACEUTICAL BULLETIN, Vol. 28, No. 12, 1980<br>Tokyo<br>T. KINOSHITA et al."A Fluorophotometric Determination of Serum Creatinine and Creatine using a Creatinineamidohydrolase-Creatineamidinohydrolase-Sarcosine Oxidase-Peroxidase System and Diacetyldichlorofluorescin"<br>pages 3501 to 3506<br>* page 3501; page 3505, chapter: "discussion" to page 3506 *<br>-- | 1-4, 8 | C 12 Q 1/34<br>C 12 Q 1/00<br>G 01 N 33/70 |
| | JOURNAL OF ANALYTICAL CHEMISTRY OF U.S.S.R., Vol. 35, No. 8, Part 2, August 1980<br>New York<br>I.F. DOLMANOVA et al. "Enzymatic Methods of Analysis"<br>pages 1042 to 1081 | | TECHNICAL FIELDS SEARCHED (Int.Cl. 3)<br>C 12 Q 1/00<br>G 01 N 31/00<br>G 01 N 33/00 |
| X | * pages 1046, 1054, 1059 * | 5 | |
| Y | * pages 1046, 1054, 1059 *<br>-- | 6,7 | |
| | DE - A1 - 3 003 490 (MILLIPORE CORP.) | | |
| X | * claims 17, 18, 21; page 9, line 34; page 10, lines 31,32 * | 5 | |
| Y | * page 10, lines 31, 32 *<br>& GB - A - 2 043 891<br>-- ./.. | 6,7 | |

CATEGORY OF CITED DOCUMENTS

X: particularly relevant if taken alone
Y: particularly relevant if combined with another document of the same category
A: technological background
O: non-written disclosure
P: intermediate document
T: theory or principle underlying the invention
E: earlier patent document, but published on, or after the filing date
D: document cited in the application
L: document cited for other reasons
&: member of the same patent family, corresponding document

X The present search report has been drawn up for all claims

| Place of search | Date of completion of the search | Examiner |
|---|---|---|
| Berlin | 28-07-1982 | HOFMANN |

0066552

European Patent Office

# EUROPEAN SEARCH REPORT

Application number

EP 82 83 0126.7

- page 2 -

| DOCUMENTS CONSIDERED TO BE RELEVANT | | | CLASSIFICATION OF THE APPLICATION (Int. Cl.3) |
|---|---|---|---|
| Category | Citation of document with indication, where appropriate, of relevant passages | Relevant to claim | |
| P,Y | EP - A1 - 0 033 462 (ELVI S.p.A.)<br>* claim 2; page 1, lines 1 to 6; page 2, lines 9 to 12; page 4, lines 1 to 3; page 5, lines 26 to 27; page 6, lines 17 to 23 *<br>---- | 6 | |
| | | | TECHNICAL FIELDS SEARCHED (Int. Cl.3) |